# FASCICULE DE BREVET EUROPEEN

(11) **EP 2 547 199 B1**
(45) Date de publication et mention de la délivrance du brevet: **16.05.2018**
(21) Numéro de dépôt: 11708853.4
(22) Date de dépôt: 16.03.2011
(51) Int. Cl.: A01N 25/02, C07C 235/74, C08K 5/20

(54) **NOUVELLES UTILISATIONS DE COMPOSES DE TYPE ESTERAMIDE**
NEUARTIGE VERWENDUNG VON ESTERAMIDVERBINDUNGEN
NOVEL USES OF ESTERAMIDE COMPOUNDS

(30) Priorité: 18.03.2010 FR 1051935
(43) Date de publication de la demande: 23.01.2013
(73) Titulaire: Rhodia Operations, 93306 Aubervilliers (FR)
(72) Inventeur: GUGLIERI, Massimo, 98000 Monaco (MC); VIDAL, Thierry, 69003 Lyon (FR)
(74) Mandataire: Cardon, Flavie
(86) Numéro de dépôt international: PCT/EP2011/053944
(87) Numéro de publication internationale: WO 2011/113852

(56) Documents cités:
- WO-A1-2009/092795
- DD-A1- 132 430
- DE-B- 1 040 234
- GB-A- 1 455 617
- US-A- 2 494 875
- US-A- 3 511 804
- US-A- 3 544 529
- US-A- 4 020 099
- US-A- 4 209 524
- SLISKOVIC D R ET AL: "Inhibitors of acyl-CoA: cholesterol O-acyl transferase (ACAT) as hypocholesterolemic agents. The synthesis and biological activity of a series of malonester amides", BIOORGANIC & MEDICINAL CHEMISTRY LETTERS, PERGAMON, ELSEVIER SCIENCE, GB LNKD- DOI:10.1016/0960-894X(96)00098-4, vol. 6, no. 6, 19 mars 1996 (1996-03-19), pages 713-718, XP004135011, ISSN: 0960-894X
- BOX V G S ET AL: "THE SYNTHESI OF BETA-LACTONES AND BETA-LACTAMS FROM MALONATES AND MALONAMIDES", HETEROCYCLES, ELSEVIER SCIENCE PUBLISHERS B.V. AMSTERDAM, NL, vol. 32, no. 2, 1 janvier 1991 (1991-01-01), pages 245-251, XP008020230, ISSN: 0385-5414
- A. ERMILI, A. CASTRO, P. WESTFALL: "Products from Attempted Vilsmeier-Haack Acylations of Pyrroles with Select Amides", JOURNAL OF ORGANIC CHEMISTRY, vol. 30, no. 2, février 1965 (1965-02), pages 339-343, XP002604984, ISSN: 0022-3263
- A. GOETA, M. SALTER, H. SHAH: "New indium-mediated cyclisation reactions of tethered haloenynes in aqueous solvent systems", TETRAHEDRON, vol. 62, 2006, pages 3582-3599, XP002604985, ISSN: 0040-4020
- BELL ET AL: "A one-pot conversion of cyclic anhydrides to ethyl .omega.-(dialkylamino)alkanoates", SYNTHETIC COMMUNICATIONS, TAYLOR & FRANCIS GROUP, PHILADELPHIA, PA LNKD- DOI:10.1080/00397918708057809, vol. 17, no. 16, 1 janvier 1987 (1987-01-01), pages 1965-1970, XP009104475, ISSN: 0039-7911
- GIORGIO ROMA ET AL: "14 H -Naphtho[1',2':5,6] pyrano[2,3- b ]quinoline derivatives", JOURNAL OF HETEROCYCLIC CHEMISTRY, vol. 12, no. 6, 1 décembre 1975 (1975-12-01), pages 1103-1109, XP55010610, ISSN: 0022-152X, DOI: 10.1002/jhet.5570120604
- H.-J. GAIS ET AL: "Acetylene mit Elektronendonator und Elektronenakzeptorgruppen", HELVETICA CHIMICA ACTA, vol. 52, no. 8, 1 janvier 1969 (1969-01-01), pages 2641-2657, XP55010669, ISSN: 0018-019X, DOI: 10.1002/hlca.19690520845
- KIM ET AL.: "The highly enantioselective phase-transfer catalytic mono-alkylation of malonamic esters", CHEMICAL COMMUNICATIONS, 14 janvier 2009 (2009-01-14), pages 782-784, XP002662345,
- DAVIES ET AL: "Preparation of N-heterocycles by radical cyclisation of enamides mediated by manganese(III) or copper (I). A comparison of cyclisation methods", TETRAHEDRON, vol. 56, 2000, pages 3941-3949, XP002662346,
- TAYLOR ET AL: "A new route to 7-substituted derivatives of N-(4-[2-(2-amino-3,4-dihydro-4-oxo-7H-pyrr olo[2,3-d]pyrimidin-5-yl)-ethyl]benzoyl)-L -glutamic acid [ ALIMTA (LY231514, MTA)]", JOURNAL OF ORGANIC CHEMISTRY, vol. 66, 2001, pages 3726-3738, XP002662347,
- BISARO ET AL: "Alkylation of active methylenes via benzhydryl cations", SYNLETT, 2002, pages 1823-1826, XP002662348,
- D'ANNIBALE ET AL: "Ceric ammonium nitrate promoted free radical cyclization reactions leading to beta-lactams", TETRAHEDRON LETTERS, vol. 38, no. 10, 1997, pages 1829-1832, XP002662349,
- D'ANNIBALE ET AL: "Mn(III)-Promoted cyclization of enamides: an oxidative radical approach to beta-lactams", TETRAHEDRON LETTERS, vol. 36, no. 49, 1995, pages 9039-9042, XP002662350,
- Andrzej Manikowski ET AL: "Facile and Versatile Room-Temperature Synthesis of N,N-Disubstituted Cyanoacetamides from Malonic Ester Chloride", Synthetic Communications, vol. 39, no. 20, 18 September 2009 (2009-09-18), pages 3621-3638, XP55195954, ISSN: 0039-7911, DOI: 10.1080/00397910902788216
- Nagatoshi Nishiwaki ET AL: "Transacylation of [alpha]-Aryl-[beta]-keto Esters", The Journal of Organic Chemistry, vol. 68, no. 22, 1 October 2003 (2003-10-01), pages 8650-8656, XP55196035, ISSN: 0022-3263, DOI: 10.1021/jo0344642

## Description

La présente invention a pour objet de nouvelles utilisations, notamment à titre de solvant, de composés de type esteramide oxalique. L'industrie utilise de nombreux composés chimiques à titre de solvants, par exemple pour préparer des produits chimiques et des matériaux, pour formuler des composés chimiques, ou pour traiter des surfaces. Par exemple, des solvants sont utilisés pour la formulation d'actifs phytosanitaires notamment sous forme de concentrés émulsionnables (Emulsifiable Concentrate "EC") destinés à être dilués dans de l'eau par l'exploitant agricole, avant application sur un champ.

L'industrie est à la recherche de nouveaux composés permettant de varier ou d'optimiser les produits et procédés dans lesquels des solvants, notamment des solvants polaires ou faiblement polaires, sont à utiliser. L'industrie a notamment besoin de composés de coût modeste, présentant des propriétés d'usage intéressantes. L'industrie a également besoin de composés présentant un profil toxicologique et/ou écologique perçu comme favorable, notamment une faible volatilité (faible VOC), une bonne biodégradabilité, une faible toxicité et/ou une faible dangerosité.

On connaît l'utilisation comme solvants des dialkylamides. Il s'agit de produits de formule R-CONMe₂, où R est un groupe hydrocarboné comme un alkyle, typiquement en C₆-C₃₀. De tels produits sont notamment commercialisés sous la dénomination Genagen® par la société Clariant. Ces solvants trouvent des applications notamment dans le domaine phytosanitaire.

On connaît également comme solvants les diesters d'acides dicarboxyliques, notamment les diesters obtenus par estérification d'un mélange d'acide adipique, d'acide glutarique et d'acide succinique. De tels produits sont notamment commercialisés sous les dénominations Rhodiasolv® RPDE, Rhodiasolv® IRIS et Rhodiasolv® DIB par la société Rhodia.

La demande internationale WO 2009/092795 décrit des composés esteramides de formule R¹OOC-A-CONR²R³, où A est un groupe alkyle comprenant de 2 à 12 atomes de carbone.

Il demeure un besoin, comme expliqué plus haut, pour de nouveaux solvants, notamment dans les formulations phytosanitaires. La présente invention a donc pour but de fournir de nouveaux solvants, particulièrement adaptés pour les applications phytosanitaires.

La présente invention concerne l'utilisation d'un composé esteramide, seul ou en mélange, de formule (I)* dans laquelle :

*R¹OOC-A-CONR2R³ (I)

- A est une liaison covalente ;
- R¹ est un groupe alkyle comprenant de 1 à 15 atomes de carbone ou un groupe cycloalkyle comprenant de 5 à 8 atomes de carbone,
- R² et R³, identiques ou différents, sont des groupes alkyles comprenant de 1 à 15 atomes de carbone,
à titre de solvant ou co-solvant.

Dans la présente demande le terme "solvant" est entendu dans un sens large, couvrant notamment les fonctions de co-solvant, d'inhibiteur de cristallisation, de décapant. Le terme solvant peut notamment désigner un produit liquide à la température d'utilisation, de préférence de point de fusion inférieur ou égal à 40°C, de préférence à 20°C, pouvant contribuer à rendre liquide une matière solide, à rendre plus fluide un liquide visqueux ou à empêcher ou à retarder la solidification ou la cristallisation de matière dans un milieu liquide.

Par co-solvant, on entend que d'autres solvants peuvent lui être associés.

L'utilisation à titre de solvant ou de co-solvant comprend notamment l'utilisation pour dissoudre un composé dans une formulation, dans un milieu réactionnel, l'utilisation pour solubiliser totalement ou partiellement un produit à éliminer (dégraissage, décapage), et/ou pour faciliter de décollage de films de matières.

Par agent d'augmentation de l'activité biologique, on désigne un composé qui, en association avec une molécule présentant une activité biologique, va permettre d'augmenter l'activité biologique de ladite molécule (par exemple synergie).

Dans la présente demande un "composé de l'invention" désigne tout composé répondant à la formule générale (I). On mentionne que le terme "composé" couvre également des mélanges de plusieurs molécules répondant à la formule générale (I). Il peut donc s'agir d'une molécule de formule (I) ou d'un mélange de plusieurs molécules de formule (I).

Dans la présente demande, par "composition de matière", on entend une composition, plus ou moins complexe, comprenant plusieurs composés chimiques. Il peut s'agir typiquement d'un produit de réaction non purifié ou modestement purifié. Le composé de l'invention pourra notamment être isolé et/ou commercialisé et/ou utilisé sous forme d'une composition de matière le comprenant. Si le composé de l'invention est un mélange de plusieurs composés de formule (I) alors c'est aussi une composition de matière. Le composé de l'invention, sous forme de molécule pure ou sous forme d'un mélange répondant à la formule (I), peut être compris dans une composition de matière.

Dans la composition de matière le composé de l'invention peut par exemple représenter au moins 10% en poids. De préférence, il s'agit du composé principal de la composition de matière. Par composé principal, on entend dans la présente demande, le composé dont la teneur est la plus élevée, même si sa teneur est inférieure à 50% en poids (par exemple dans un mélange de 40% de A, de 30% de B, et de 30% de C, le produit A est le composé principal). Encore plus préférablement le composé de l'invention représente au moins 50% en poids de la composition de matière, par exemple de 70% à 95% en poids, et même de 75% à 90% en poids. Comme indiqué plus haut, la composition de matière peut être un produit de réaction.

Les composés de l'invention répondent à la formule (I).

Dans la formule (I), le groupe R¹ est un groupe alkyle comprenant de 1 à 15 atomes de carbone ou un groupe cycloalkyle comprenant de 5 à 8 atomes de carbones, les groupes R² et R³, identiques ou différents, sont des groupes alkyles comprenant de 1 à 15 atomes de carbone. Comme exemples préférés de groupes carbocycliques et monocycliques pour R¹, on peut citer les groupes cyclopentyle, cyclohexyle, cyclohexènyle ou cyclopentadiènyle.

Selon la présente invention, les groupes "alkyle" représentent des groupes hydrocarbonés saturés, en chaîne droite ou ramifiée, comprenant de 1 à 15 atomes de carbone (ils peuvent typiquement être représentés par la formule CₙH₂ₙ₊₁, n étant un nombre entier représentant le nombre d'atomes de carbone).

On peut notamment citer, lorsqu'ils sont linéaires, les groupes méthyle, éthyle, propyle, butyle, pentyle, hexyle, heptyle, octyle, nonyle, décyle, undécyle, dodecyle, tridécyle, tetradécyle et pentadécyle. On peut notamment citer, lorsqu'ils sont ramifiés ou substitués par un ou plusieurs groupes alkyle, les groupes isopropyle, isobutyle, tert-butyle, sec-butyle, isopentyle, 2-méthylbutyle, sec-pentyle, isohexyle, sec-hexyle, 2-éthylbutyle, 3-méthylpentyle, isoheptyle, sec-heptyle, 3-méthylhexyle, 4-méthylhexyle, 1-éthylpentyle, 2-éthylpentyle, 3-éthylpentyle, isooctyle, et 3-méthylheptyle.

Par groupe "cycloalkyle", on envisage plus particulièrement un groupe carbocyclique monocyclique ayant de 5 à 8 atomes de carbone, et de préférence 5 ou 6 atomes de carbone. On peut mentionner préférentiellement le groupe cyclopentyle ou cyclohexyle.

De façon avantageuse, la présente invention concerne l'utilisation telle que définie ci-dessus, d'un composé de formule (I) dans laquelle R¹ est choisi parmi les groupes méthyle, éthyle, n-propyle, isopropyle, n-butyle, isobutyle, tertiobutyle, sec-butyle, n-pentyle, isopentyle, sec-pentyle, cyclopentyle, n-hexyle, isohexyle, sec-hexyle, cyclohexyle, méthylcyclohexyle, 2-éthylbutyle, 3-méthylpentyle, n-heptyle, isoheptyle, sec-heptyle, 3-méthylhexyle, 4-méthylhexyle, 1-éthylpentyle, 2-éthylpentyle, 3-éthylpentyle, n-octyle, isooctyle, 3-méthylheptyle, n-nonyle, n-décyle, undécyle, n-dodécyle, tridécyle, tétradécyle et pentadécyle.

Selon un autre mode de réalisation avantageux, la présente invention concerne l'utilisation telle que définie ci-dessus, d'un composé de formule (I) dans laquelle R² et R³, identiques ou différents, sont choisis parmi les groupes méthyle, éthyle, n-propyle, isopropyle, n-butyle, isobutyle, tertiobutyle, n-pentyle, isoamyle et hexyle.

De façon particulièrement avantageuse, la présente invention concerne l'utilisation telle que définie ci-dessus, d'un composé de formule (I) dans laquelle R² et R³ sont des groupes méthyle et R¹ est choisi parmi les groupes méthyle, éthyle, n-propyle, isopropyle, n-butyle, isobutyle, tertiobutyle, sec-butyle, n-pentyle, isopentyle, sec-pentyle, cyclopentyle, n-hexyle, isohexyle, sec-hexyle, cyclohexyle, méthylcyclohexyle, 2-éthylbutyle, 3-méthylpentyle, n-heptyle, isoheptyle, sec-heptyle, 3-méthylhexyle, 4-méthylhexyle, 1-éthylpentyle, 2-éthylpentyle, 3-éthylpentyle, n-octyle, isooctyle, 3-méthylheptyle, n-nonyle, n-décyle, undécyle, n-dodécyle, tridécyle, tétradécyle et pentadécyle.

Selon un autre mode de réalisation avantageux, R¹ est un groupe méthyle et R² et R³ sont différents et tels que définis ci-dessus.

Selon un autre mode de réalisation avantageux, R² et R³ sont identiques et sont choisis parmi les groupes méthyle, éthyle, n-propyle, isopropyle, et isooctyle, et R¹ est choisi parmi les groupes méthyle, éthyle, propyle, isopropyle, n-butyle, isobutyle, tertiobutyle.

Selon un autre mode de réalisation avantageux, R¹ est un groupe méthyle et R² et R³ sont différents et choisis parmi les groupes méthyle, éthyle, propyle, isopropyle, n-butyle, tertiobutyle.

Parmi les composés préférés mis en oeuvre dans le cadre de l'utilisation selon la présente invention, on peut notamment citer les composés suivants :
- CH₃OOC-CONMe₂ ;
- CH₃CH₂OOC-CONMe₂ ;
- CH₃-CH₂-CH₂-CH₂-CH(CH₂CH₃)-CH₂-OOC-CONMe₂ ;
- CH₃-(CH₂)₉-OOC-CONMe₂ ;
- CH₃-(CH₂)ₙ-OOC-CONMe₂ ;
- C₆H₁₁-OOC-CONMe₂ ;
- CH₃OOC-CONEt₂ ;
- CH₃CH₂OOC-CONEt₂ ;
- CH₃-CH₂-CH₂-CH₂-CH(CH₂CH₃)-CH₂-OOC-CONEt₂ ;
- CH₃-(CH₂)₉-OOC-CONEt₂;
- CH₃-(CH₂)₁₁-OOC-CONEt₂;
- C₆H₁₁-OOC-CONEt₂.

De préférence, les composés mis en oeuvre dans le cadre de la présente invention présentent un point de fusion inférieur à ou égal à 40°C, de préférence à 20°C.

Certains composés sont connus ou disponibles dans le commerce. Dans le cas contraire, ils peuvent obtenus selon le procédé décrit ci-après.

Le procédé de préparation des composés esteramide de formule (I) telle que définie ci-dessus comprend les étapes suivantes :
a) la réaction d'un composé de formule (II) ROOC-A-COOR, R étant un groupe méthyle ou un groupe éthyle, avec une amine de formule HNR²R³, R² et R³ étant tels que définis ci-dessus dans la formule (I), en présence d'un catalyseur basique, pour obtenir un composé de formule (III) suivante : ROOC-A-CONR²R³ ; et
b) la réaction du composé de formule (III) susmentionnée avec un alcool de formule R¹OH, R¹ étant tel que défini ci-dessus dans la formule (I), en présence d'un catalyseur, pour obtenir le composé de formule (I).

Les composés de la présente invention peuvent être également préparés selon le procédé comprenant les étapes suivantes :
a') la réaction d'un composé de formule (II) ROOC-A-COOR, R étant un groupe méthyle ou un groupe éthyle, avec un alcool de formule R¹OH, R¹ étant tel que défini ci-dessus dans la formule (I), en présence d'un catalyseur, pour obtenir un mélange constitué du composé de formule (IV) suivante : R¹OOC-A-COOR et du composé de formule (V) suivante : R¹OOC-A-COOR¹ ; et
b') la réaction du mélange susmentionné tel qu'obtenu à l'étape précédente a') avec une amine de formule HNR²R³, R² et R³ étant tels que définis ci-dessus dans la formule (I), en présence d'un catalyseur basique, pour obtenir le composé de formule (I).

Les composés de la présente invention peuvent être également préparés selon le procédé comprenant les étapes suivantes :
a") la réaction d'un composé de formule (II) ROOC-A-COOR, R étant un hydrogène ou un groupe méthyle ou un groupe éthyle, avec un alcool de formule R¹OH, R¹ étant tel que défini ci-dessus dans la formule (I), en présence d'un catalyseur acide, pour obtenir un mélange constitué du composé de formule (IV) suivante : R¹OOC-A-COOR et du composé de formule (V) suivante : R¹OOC-A-COOR¹ ; et
b") la réaction du mélange susmentionné tel qu'obtenu à l'étape précédente a') avec une amine de formule HNR²R³, R² et R³ étant tels que définis ci-dessus dans la formule (I), en présence d'un catalyseur basique, pour obtenir le composé de formule (I).

Parmi les catalyseurs basiques pouvant être mis en oeuvre dans les étapes a), b') et b"), on peut citer notamment le méthylate de sodium (NaOMe), l'éthylate de sodium (NaOEt), le sodium (Na), le carbonate de potassium (K₂CO₃), le carbonate de sodium (Na₂CO₃), l'hydrure de sodium (HNa), l'amidure de sodium (NH₂Na) ou les suppports solides basiques tels que des résines basiques, des alumines.

Pour la mise en oeuvre des étapes b) et a'), on peut utiliser indifféremment un catalyseur basique ou un catalyseur acide.

Parmi les catalyseurs acides, on peut notamment citer les acides de Lewis ou de Brönsted, et plus particulièrement l'acide sulfurique (H₂SO₄), le tétrabutoxyde de titane (IV) (Ti(OBu)₄), les résines acides (par exemple sulfoniques), l'éthérate de trifluorure de Bore (BF₃OEt2), le chlorure d'aluminium (AlCl₃), le chlorure de Zinc (ZnCl₂), les dérivés de terres rares tel que le chlorure de Lanthane (LaCl₃), le chlorure d'étain (SnCl₂), de bismuth (BiCl₃), ou l'acétate de dibutylétain (Bu₂SnOAc).

En ce qui concerne les catalyseurs basiques, ils sont également choisis parmi les catalyseurs mentionnés ci-dessus pour les étapes a), b') et b").

Pour toutes les étapes du procédé de l'invention, il est également possible d'utiliser un catalyseur qui est une enzyme supportée du type amidase ou lipase, de telles enzymes étant disponibles commercialement.

Les étapes a), b') et b") du procédé de l'invention sont avantageusement conduites à une température comprise entre 20 et 150°C, de préférence entre 20 et 100°C et notamment entre 20 et 50°C. La pression est généralement comprise entre 1 et 5 bars.

Les étapes a'), a") et b) du procédé de l'invention sont avantageusement conduites à une température comprise entre 20 et 180°C, de préférence entre 60 et 160°C et notamment entre 100 et 150°C. La pression est généralement proche de la pression atmosphérique.

Les étapes a), a'), a"), b), b') et b") sont avantageusement conduites sous atmosphère de gaz inerte, de préférence sous inertage d'azote.

Les conditions de concentration des réactifs introduits dans le procédé de l'invention sont avantageusement choisies de telle sorte que le milieu réactionnel est homogène, tout en limitant la dilution du milieu réactionnel pour des raisons écologiques et économiques.

Les produits issus du procédé de l'invention sont récupérés selon des méthodes classiques connues de l'homme du métier, comme par exemple au moyen d'une étape de distillation, de filtration ou d'extraction.

Le composé de formule (II) ROOC-A-COOR, R étant méthyle ou éthyle, est préparé par réaction de l'acide oxalique HOOC-COOH avec respectivement le méthanol ou l'éthanol, de préférence en présence d'un catalyseur acide tel que l'acide sulfurique et tout en éliminant l'eau formée en cours de réaction.

La présente invention concerne également l'utilisation telle que définie ci-dessus, dans laquelle le composé de formule (I) est dans une formulation phytosanitaire, dans une formulation de nettoyage, dans une formulation de décapage, dans une formulation de dégraissage, dans une formulation de lubrifiant, dans une formulation de revêtement, dans une formulation de pigment ou encre, dans une formulation de solubilisation des résines, notamment des résines PVDF (poudre du fluorure de polyvinylidène), dans une formulation de nettoyage des « photoresists » ou encore dans une formulation de nettoyage d'écrans, notamment d'écrans à cristaux liquides (LCD).

Le composé peut par exemple être utilisé à titre d'agent de coalescence dans une formulation de peinture aqueuse.

Le composé peut notamment être utilisé comme solvant de résines par exemple dans l'industrie du revêtement de câbles ou dans l'industrie électronique, notamment comme solvant du PVDF.

Le composé peut notamment être utilisé comme solvant de nettoyage et/ou de décapage dans l'industrie électronique. Il peut notamment être utilisé dans des batteries au Lithium. Il peut notamment être utilisé sur des résines photorésistantes, des polymères, des cires des graisses, des huiles.

Le composé peut notamment être utilisé pour le nettoyage d'encres, par exemple lors de la production d'encres ou lors de l'utilisation d'encre en impression.

Le composé peut notamment être utilisé pour le blanchiement du papier.

Le composé peut notamment être utilisé pour le nettoyage de tamis ou d'autres outils mis en oeuvre dans des procédés de fabrication et/ou de recyclage de papier.

Le composé peut notamment être utilisé pour le nettoyage de bitumes ou de sables bitumineux ("tar sand" en anglais), par exemple sur les substrats revêtus, sur les outils utilisés pour appliquer ces matières, sur des vêtements souillés, sur des véhicules souillés.

Le composé peut notamment être utilisé pour le nettoyage d'engins volants comme des avions, des hélicoptères, des navettes spatiales.

Le composé peut notamment être utilisé comme agent de dégraissage sur des surfaces métalliques, par exemple des surfaces d'outils, d'objets manufacturés, de tôles, de moules, notamment en acier ou en aluminium ou en alliages de ces métaux.

Le composé peut notamment être utilisé comme solvant de nettoyage sur des surfaces dures ou des surfaces textiles.

Le composé peut notamment être utilisé comme solvant de décapage de peinture ou de résines, sur des surfaces d'outils, par exemple des moules de fonderie, sur des surfaces des sites industriels (sols, cloisons, etc...).

Le composé peut notamment être utilisé comme agent plastifiant dans des formulations de polymères thermoplastiques.

Les formulations de nettoyage et/ou de dégraissage peuvent notamment être des formulations pour les soins ménagers, opérés dans les foyers ou dans les domaines publiques (hôtels, bureaux, usines....). Il peut s'agir de formulations pour le nettoyage des surfaces dures comme les sols, les surfaces d'ameublement et d'équipement des cuisines et salles de bain, la vaisselle. Ces formulations peuvent également être utilisées dans la sphère industrielle pour dégraisser des produits manufacturés et/ou les nettoyer.

Dans le cadre d'utilisations dans des milieux réactionnels, on cite notamment les utilisations dans le cadre de polymérisations en solution, notamment pour la préparation en solution de polycondensats, notamment de polyimides ou de polyesters ou de polyamides ou de polyamide-imides ou de polyuréthane en dispersion aqueuse (PUD), notamment de polycondensats partiellement ou totalement aromatiques comme des polyamides aromatiques (aramides).

La présente invention concerne également une formulation phytosanitaire, comprenant un composé esteramide de formule (I) suivante :

R¹OOC-A-CONR²R³ (I)

où A, R¹, R² et R³ sont tels que définis ci-dessus,
en association avec un produit actif.

Le composé de l'invention et/ou une composition de matière le comprenant décrite ci-dessus, peut notamment être utilisé dans des formulations phytosanitaires comprenant un produit actif solide ou liquide visqueux. Plus de détails sont donnés ci-dessous, où le mot "solvant" peut désigner le composé de l'invention ou une composition de matière le comprenant, décrite ci-dessus.

La formulation phytosanitaire est généralement une formulation phytosanitaire concentrée comprenant un produit actif.

L'agriculture utilise de nombreuses matières actives (ou produits actifs) telles que des fertilisants ou des pesticides, par exemple des insecticides, herbicides ou fongicides. On parle de produits phytosanitaires actifs (ou de matière active). Les produits phytosanitaires actifs sont généralement produits sous forme pure ou très concentrée. Ils doivent être utilisés sur les exploitations agricoles à de faibles concentrations ou utilisés pour traiter les produits agricoles après la recolte. A cette fin, ils sont généralement formulés avec d'autres ingrédients afin de permettre une dilution aisée par l'exploitant agricole. On parle de formulations phytosanitaires. La dilution opérée par l'exploitant agricole est généralement réalisée par mélange de la formulation phytosanitaire avec de l'eau.

Ainsi les formulations phytosanitaires doivent permettre une dilution aisée par l'exploitant agricole, afin d'obtenir un produit dans lequel le produit phytosanitaire est correctement dispersé, par exemple sous forme de solution, d'émulsion, de suspension, ou de suspo-émulsion. Les formulations phytosanitaires permettent ainsi le transport d'un produit phytosanitaire sous forme relativement concentrée, un conditionnement aisé et/ou une manipulation aisée pour l'utilisateur final. Différents types de formulations phytosanitaires peuvent être utilisés selon les différents produits phytosanitaires. On cite par exemple les concentrés émulsionnables (Emulsifiable Concentrates «EC»), les émulsions concentrées (Emulsion in water "EW"), les microémulsions ("ME"), les poudres mouillables (Wettable Powders «WP»), les granulés dispersables dans l'eau (Water Dispersible Granules, «WDG»). Les formulations qu'il est possible d'utiliser dépendent de la forme physique du produit phytosanitaire (par exemple solide ou liquide), et de ses propriétés physico-chimiques en présence d'autres composés comme l'eau ou les solvants.

Après dilution par l'exploitant agricole, par exemple par mélange avec de l'eau, le produit phytosanitaire peut se trouver sous différentes formes physiques : solution, dispersion de particules solides, dispersion de gouttelettes du produit, gouttelettes de solvant dans lequel le produit est dissous... Les formulations phytosanitaires comprennent généralement des composés permettant d'obtenir ces formes physiques. Il peut par exemple s'agir de tensioactifs, de solvants, de supports minéraux, et/ou de dispersants. Bien souvent ces composés n'ont pas un caractère actif, mais un caractère d'ingredient d'aide à la formulation. Les formulations phytosanitaires peuvent notamment être sous forme liquide, ou sous forme solide.

Afin de préparer des formulations phytosanitaires de produits phytosanitaires actifs solides, il est connu de solubiliser le produit dans un solvant. La formulation phytosanitaire comprend ainsi une solution du produit dans le solvant. La formulation peut être sous forme solide, par exemple sous forme de poudre mouillable (WP) où la solution imbibe un support inorganique, par exemple du kaolin et/ou de la silice. La formulation peut alternativement être sous forme liquide, par exemple sous forme de concentré émulsionnable (EC) présentant une seule phase liquide limpide comprenant le solvant et le produit en solution, pouvant former une émulsion par ajout d'eau, sans agitation ou avec une faible agitation. Elle peut aussi être sous forme d'une émulsion concentrée (EW), dont la phase dispersée dans l'eau comprend le solvant et le produit en solution dans le solvant. Elle peut aussi être sous forme d'une microémulsion (ME), limpide, dont la phase dispersée dans l'eau comprend le solvant et le produit en solution dans le solvant, de concentré soluble (SL) presentant une seule phase liquide limpide comprenant le solvant et le produit en solution, pouvant former une solution par ajout d'eau, ou de suspo-emulsion (SE), contenant au moins deux phases en dispersion, une solide et une liquide.

Certains actifs phytosanitaires solides sont souvent difficiles à formuler. Pour certains actifs phytosanitaires, il est difficile de réaliser des formulations concentrées, faciles à diluer pour l'exploitant agricole, stables, et sans inconvénients (avérés ou perçus) substantiels en matière de sécurité, de toxicité et/ou d'eco-toxicité. Pour certains actifs, il est difficile de formuler à des concentrations relativement élevées, avec une stabilité suffisante. En particulier il est nécessaire d'éviter l'apparition de cristaux en particulier à basse température et/ou lors de la dilution et/ou lors du stockage de la composition diluée. Les cristaux peuvent avoir des effets négatifs, notamment boucher les filtres des dispositifs utilisés pour répandre la composition diluée, boucher les dispositifs de pulvérisation, diminuer la quantité de formulation distribuée sur le champ, créer des problèmes inutiles de filières de déchets pour éliminer les cristaux, et/ou provoquer une mauvaise répartition du produit actif sur le champ agricole. Par exemple le tebuconazole est un fongicide particulièrement efficace, et d'utilisation répandue, pour la culture du soja notamment qui montre souvent ce type de comportement.

Les formulations comprenant le solvant de la présente invention présentent notamment :
- une solubilisation de quantités importantes d'actifs,
- une absence de cristallisation, même des conditions exigeantes, et/ou
- une bonne activité biologique pouvant être due à une bonne solvatation.

La formulation phytosanitaire peut en outre être une formulation phytosanitaire concentrée comprenant :
a) un produit phytosanitaire actif,
b) le composé esteramide de formule (I) selon la présente invention,
c) éventuellement au moins un co-solvant ou un autre solvant,
d) éventuellement au moins agent tensioactif,
e) éventuellement de l'eau.

Des produits phytosanitaires actifs, notamment des produits non solubles dans l'eau et solides sont connus de l'homme du métier. Le produit phytosanitaire actif peut notamment être un herbicide, un insecticide, un acaricide, un fongicide, ou un agent d'élimination des rongeurs ("rodenticide" en anglais) par exemple un raticide.

Comme exemples d'insecticides et acaricides convenant à l'invention, on peut citer ceux qui appartiennent aux familles :
- des organo-halogénés ou chlorés tels que par exemple le D.D.T. (dichloro diphényl trichloro-éthane), le lindane (isomère γ de l'hexachloro-cyclohexane), le chlordane (octachlorotétrahydro méthano indène), le toxaphène ;
- des carbinols tels que par exemple le dicofol (dichlorophényl trichloroéthanol) ;
- des organophosphorés tels que par exemple le bromophos [(4-bromo-2,5-dichloro-phenoxy)-dimethoxy-thioxo-phosphorane), le diazinon (O,O-diéthyl-O-(2-isopropyl-6-méthyl-pyrimidin-4-yl)phosphorothioate), le féni-trothion (O,O-diméthyl-O-nitro-4-m-tolylphosphorothioate), le malathion (S-1,2-bis(éthoxycarbonyl)éthyl-O,O-diméthyl-phosphorodithioate), le parathion (O,O-diéthyl-O-nitro-4-phénylphosphorothioate), le trichlorfon (diméthyl-2,2,2-trichloro-1-hydroxy-éthylphosphonate], le diméthoate (O,O-diméthyl-S-méthylcarbamoylméthyl phosphorodithioate) ;
- des sulfones et sulfonates tels que par exemple le tétradifon (tétrachloro diphénylsulfone) ;
- des carbamates tels que par exemple le carbaryl (N-méthylcarbamate de naphtyle), le méthomyl (N-méthylcarbamate de (méthylthio éthylidène amine)) ;
- des benzoylurées tel que par exemple le diflubenzuron (difluoro benzoyl chlorophénylurée] ;
- les pyrethrinoides de synthèse ;
- les acaricides tels que par exemple le cyhéxatin (tricyclohexyl-hydroxystannane).

Les fongicides susceptibles d'être mis en oeuvre dans l'invention peuvent par exemple être choisis parmi :
- les carbamates comme par exemple le bénomyl (butylcarbamoyl benzimidazolyl carbamate de méthyle), le carbendazime (benzimidazolyl carbamate de méthyle), le zirame (diméthyl dithiocarbamate de zinc), le zinèbe (ethylène-bis dithiocarbamate de zinc), le manèbe (ethylène-bis dithiocarbamate de manganèse), le mancozèbe (éthylène-bis dithiocarbamate de zinc et de manganèse), le thirame (disulfure de bis diméthyl-thiocarbamoyle) ;
- les dérivés du benzène comme par exemple le PCNB (pentachloronitrobenzène) ;
- les dérivés du phénol comme par exemple le dinocap (crotonate de (méthylheptyl)dinitrophényl) ;
- les quinones comme par exemple le dithianon (dioxodihydro naphto dithiine dicarbonitrile) ;
- les dicarboximides comme par exemple le captane (trichlorométhylthio tétrahydroisoindolinedione), le folpel (trichlorométhylthio isoindolinedione), l'iprodione (dichlorophényl isopropyl carbamoyl dichlorophényl-hydantoïne) ;
- les amines et amides comme par exemple le bénodanil (iodobenzanilide), le métalaxyl (diméthylphényl méthoxyacétyl alalinate de méthyle) ;
- les diazines comme par exemple le pyrazophos (thiophosphate d'éthyle et d'éthoxycarbonyl méthyl pyrazolo pyrimidine), le fénarimol (chlorophényl chlorophényl pyrimidine méthanol) ;
- les sulfamides et dérivés soufrés comme par exemple le dichlofluanide (dichloro fluoro méthylthiodiméthyl phényl sulfamide) ;
- les guanidines comme par exemple la doguadine (acétate de dodécylguanidine) ;
- les hétérocycles comme par exemple l'étridiazole (éthoxy trichlorométhyl thiadiazole), le triadiméfon (chlorophénoxy diméthyltriazol butanone) ;
- les monoéthyl phosphites métalliques comme par exemple le phoséthyl-Al (tris-O-éthylphosphonate d'aluminium) ;
- les organostanniques comme par exemple le fentine-acétate (triphényl étain].

A titre de substances chimiques présentant des propriétés herbicides, on peut faire appel à ceux qui se retrouvent dans les formules chimiques suivantes :
- les composés phénoliques tels que, par exemple, le dinosèbe (dinitrobutylphénol) ;
- les carbamates tels que, par exemple, le phenmédiphame (tolylcarbamoyloxyphényl carbamate de méthyle) ;
- les urées substituées tels que, par exemple, le néburon (butyl dichlorophényl méthyl urée), le diuron (dichlorophényl diméthyl urée), le linuron (dichlorophényl méthoxyméthyl urée) ;
- les diazines tels que, par exemple, le bromacil (bromobutyl méthyl uracile), le chloridazone (phénylamino chloropyridazone),
- les triazines tels que, par exemple, la simazine (chloro bis-éthylamino s-triazine), l'atrazine (chloroéthylamino isopropylamino-s-triazine), la terbuthylazine (chloroéthylamino butylamino s-triazine), le terbuméton (tert-butylamino éthylamino méthoxy triazine), le prométryne (méthylthio bis isopropylamino s-triazine), l'amétryne (méthylthio éthylamino isopropylamino s-triazine), la métribuzine (méthylthio butylamino triazine-one), la cyanazine (chloro éthylamino s-triazine-ylaminométhyl-propionitrile) ;
- les amides tels que, par exemple, le napropamide (naphtoxydiéthyl propionamide), le propachlore- (isopropyl chloroacétanilide] ;
- les ammoniums quaternaires ;
- les benzonitriles ;
- les toluidines tels que, par exemple, l'éthalfluraline (dinitro-éthylméthyl propényl trifluoro méthylaniline), l'oryzalin (dinitrodipropyl sulphanil-amide) ;
- les triazoles ;
- les dérivés divers tels que, par exemple, le bénazoline ((acide chloro oxo benzothiazoline acétique), le diméfuron (chloro oxo tert-butyl oxadiazoline phényl diméthyl urée), le bromophénoxime (dibromo hydroxy dinitro phényl benzaldoxime], le pyridate (chlorophénylpyridazinylcarbothiolate octyle).

Comme autres exemples de biocides pouvant être utilisés selon l'invention on peut citer les nématicides, les molluscicides etc.. Il est possible de mettre en oeuvre une ou plusieurs matières actives appartenant à la même classe de biocides ou à une classe différente.

Ainsi, à titre d'exemples non limitatifs de matières actives préférées, on peut citer entre autres l'Amétryne, le Diuron, le Linuron, le Chlortoluron, l'Isoproturon, le Nicosulfuron, le Metamitron, le Diazinon, l'Aclonifen, l'Atrazine, le Chlorothalonil, le Bromoxynil, le Bromoxynil heptanoate, le Bromoxynil octanoate, le Mancozeb, la Manèbe, le Zineb, la Phenmédipham, le Propanyl, la série des phénoxyphénoxy, la série des hétéroaryloxyphénoxy, le CMPP, le MCPA, le 2,4-D, la Simazine, les produits actifs de la série des imidazolinones, la famille des organophosphorés, avec notamment l'Azinphos-éthyl, l'Azinphos-méthyl, l'Alachlore, le Chlorpyriphos, le Diclofop-méthyl, le Fénoxaprop-p-éthyl, le Méthoxychlore, la Cyperméthrine, le Fenoxycarbe, le cymoxanil, le chlorothalonyl, les insecticides neonicotinoides, la famille des fongicide triazoles tels que l'azaconazole, bromuconazole, cyproconazole, difenoconazole, diniconazole, epoxyconazole, fenbuconazole, flusilazole, myclobutanyl, tebuconazole, triadimefon, triadimenol, des strobilurines telles que la pyraclostrobine, la picoxystrobine, l'azoxystrobine, la famoxadone, le kresoxym-méthyl et la trifloxystrobine, les solfonylurées telles que le bensulfuron-méthyl, le chlorimuron-éthyl, le chlorsulfuron, le metsulfuron-méthyl, le nicosulfuron, le sulfomethuron-méthyl, le triasulfuron, le tribenuron-méthyl.

On choisit parmi cette liste les produits non-hydrosolubles.

On peut notamment mettre en oeuvre les produits phytosanitaires actifs suivants :

| | |
|---|---|
| Alachlor | 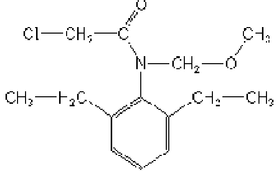 |
| Chlorpyrifos | 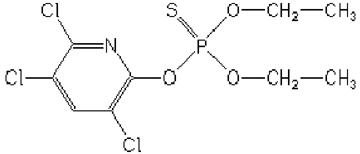 |
| alpha-cyperméthrine | 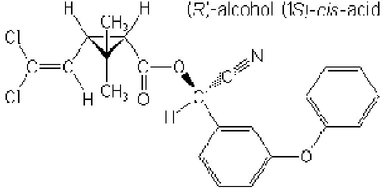 |
| | 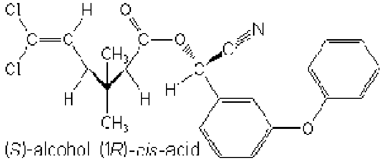 |
| | En mélange racémique et/ou en stéréoisomères isolés. |
| Phenmedipham | 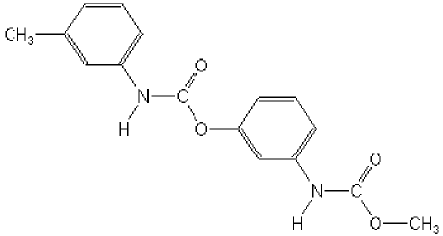 |
| Propanil | 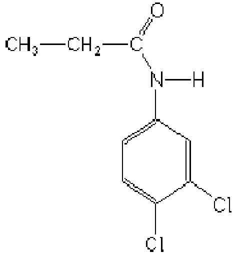 |
| Pendimethalin | 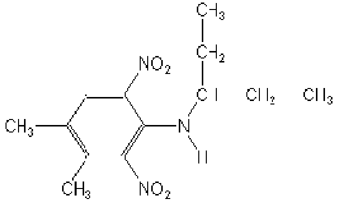 |
| triadimenol | 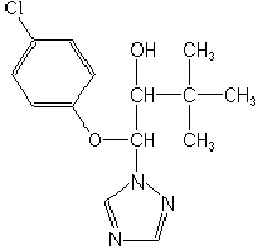 |
| Trifluralin | 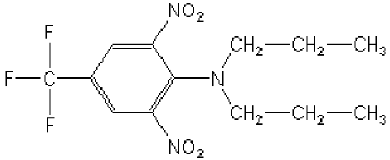 |
| Oxyfluorfen | 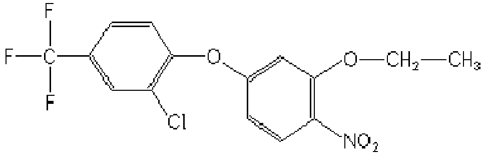 |
| Dimethoate | 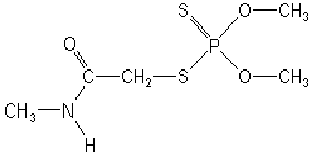 |
| Imidacloprid | 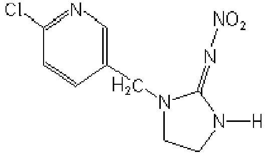 |
| Proxopur | 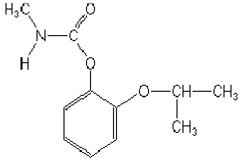 |
| Benomyl | 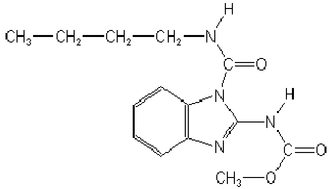 |
| Deltamethrine | 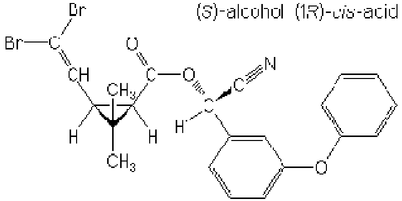 |
| Fenvalerate | 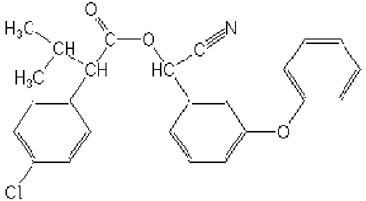 |
| Abamectin | 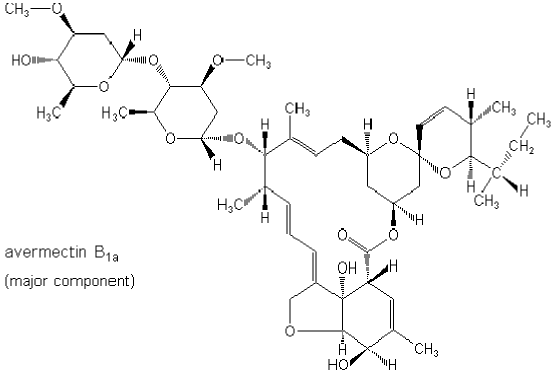 |
| | 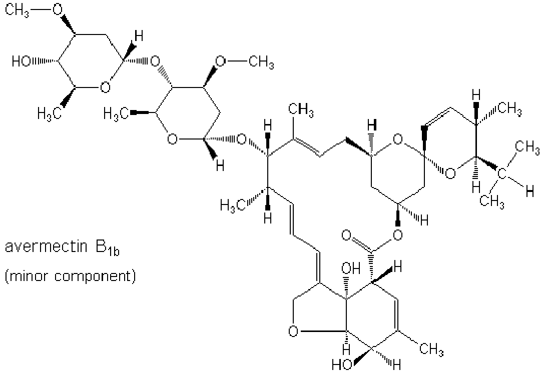 |
| Amicarbazone | 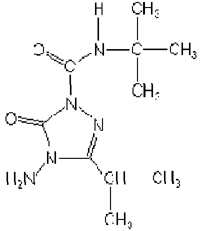 |
| Bifenthrin | 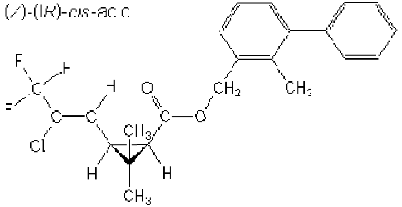 |
| | 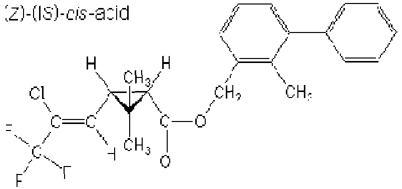 |
| Carbosulfan | 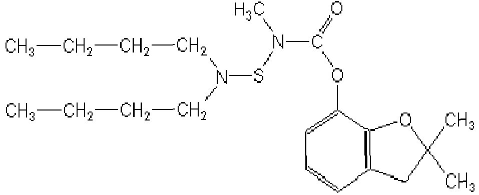 |
| Cyfluthrin | 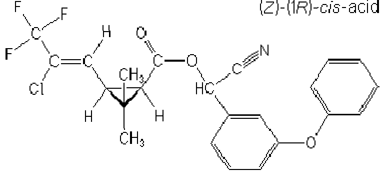 |
| | 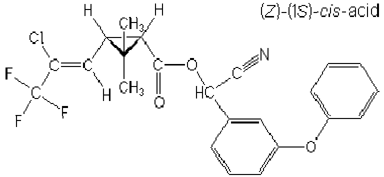 |
| Difenconazole | 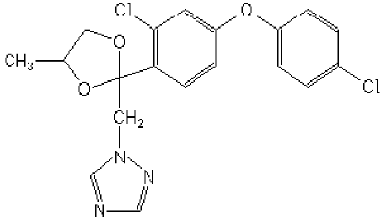 |
| Ethofenprox | 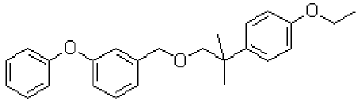 |
| Fenoxapropethyl | 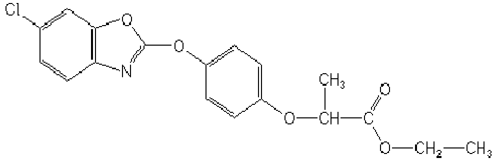 |
| Fipronil | 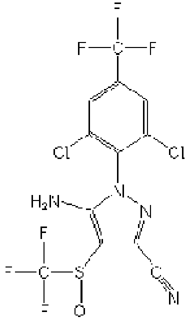 |
| Fenvalerate | 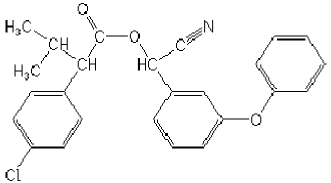 |
| Fluazifop-p-butyl | 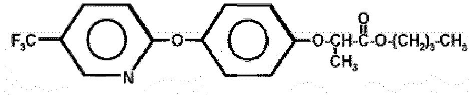 |
| Flufenouron | 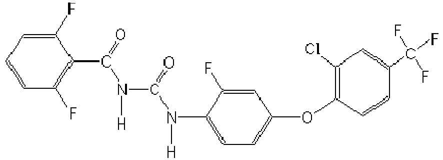 |
| Hexazinone | 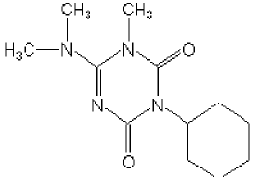 |
| Lambda-cyalothrin | 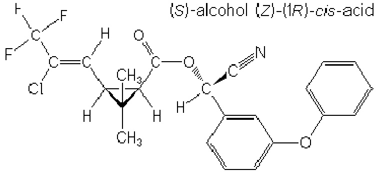 |
| | 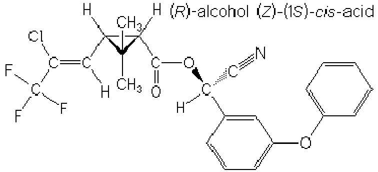 |
| Methomyl | 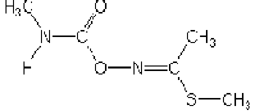 |
| Permethrin | 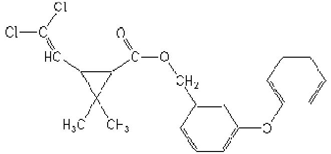 |
| Prochloraz | 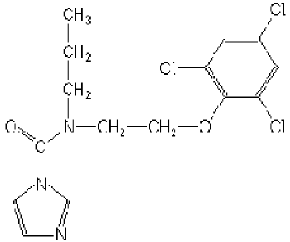 |
| Propiconazole | 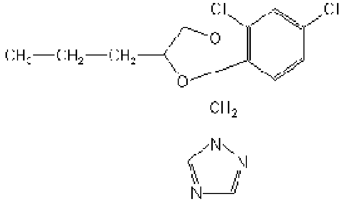 |
| Tebuconazole | 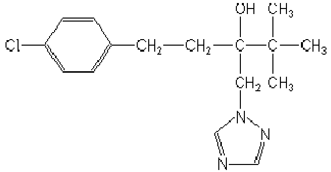 |

Ces produits et dénominations sont connus de l'homme du métier. On peut associer plusieurs produits phytosanitaires actifs.

La formulation phytosanitaire peut comprendre un agent tensioactif, de préférence un émulsifiant. Les agents émulsifiants sont des agents destinés à faciliter la mise en émulsion après mise en présence de la formulation avec de l'eau, et/ou à stabiliser (dans le temps et/ou en température) l'émulsion, par exemple en évitant une séparation des phases.

Le tensioactif peut être un tensioactif anionique, non ionique de préférence polyalkoxylé, cationique, amphotère (terme incluant aussi les tensioactifs zwitterioniques). Il peut s'agir d'un mélange ou d'une association de ces tensioactifs.

A titre d'exemples de tensioactifs anioniques, on peut citer, sans intention de s'y limiter :
- les acides alkylsulfoniques, les acides arylsulfoniques, éventuellement substitués par un ou plusieurs groupements hydrocarbonés, et dont la fonction acide est partiellement ou totalement salifiée, comme les acides alkylsulfoniques en C₈-C₅₀, plus particulièrement en C₈-C₃₀, de préférence en C₁₀-C₂₂, les acides benzènesulfoniques, les acides naphtalènesulfoniques, substitués par un à trois groupements alkyle en C₁-C₃₀, de préférence en C₄-C₁₆, et/ou alcényles en C₂-C₃₀, de préférence en C₄-C₁₆,
- les mono- ou diesters d'acides alkylsulfosucciniques, dont la partie alkyle, linéaire ou ramifiée, éventuellement substituée par un ou plusieurs groupements hydroxylés et/ou alkoxylés, linéaires ou ramifiés en C₂-C₄ (de préférence éthoxylés, propoxylés, éthopropoxylés),
- les esters phosphates choisis plus particulièrement parmi ceux comprenant au moins un groupement hydrocarboné saturé, insaturé ou aromatique, linéaire ou ramifié, comprenant 8 à 40 atomes de carbone, de préférence 10 à 30, éventuellement substitués par au moins un groupement alkoxylé (éthoxylé, propoxylé, éthopropoxylé). En outre, ils comprennent au moins un groupe ester phosphate, mono- ou diestérifié de telle sorte que l'on puisse avoir un ou deux groupes acides libres ou partiellement ou totalement salifiés. Les esters phosphates préférés sont du type des mono- et diesters de l'acide phosphorique et de mono-, di- ou tristyrylphénol alkoxylé (éthoxylé et/ou propoxylé), ou de mono-, di- ou trialkylphénol alkoxylé (éthoxylé et/ou propoxylé), éventuellement substitué par un à quatre groupements alkyle ; de l'acide phosphorique et d'un alcool en C₈-C₃₀, de préférence en C₁₀-C₂₂ alkoxylé (éthoxylé ou éthopropoxylé); de l'acide phosphorique et d'un alcool en C₈-C₂₂, de préférence en C₁₀-C₂₂, non alkoxylé,
- les esters sulfates obtenus à partir d'alcools saturés, ou aromatiques, éventuellement substitués par un ou plusieurs groupements alkoxylés (éthoxylés, propoxylés, éthopropoxylés), et pour lesquels les fonctions sulfates se présentent sous la forme acide libre, ou partiellement ou totalement neutralisées. A titre d'exemple, on peut citer les esters sulfates obtenus plus particulièrement à partir d'alcools en C₈-C₂₀, saturés ou insaturés, pouvant comprendre 1 à 8 motifs alkoxylés (éthoxylés, propoxylés, éthopropoxylés) ; les esters sulfates obtenus à partir de phénol polyalkoxylé, substitués par 1 à 3 groupements hydroxycarbonés en C₂-C₃₀, saturés ou insaturés, et dans lesquels le nombre de motifs alkoxylés est compris entre 2 et 40 ; les esters sulfates obtenus à partir de mono-, di- ou tristyrylphénol polyalkoxylés dans lesquels le nombre de motifs alkoxylés varie de 2 à 40.

Les tensioactifs anioniques peuvent être sous forme acide (il sont potentiellement anioniques), ou sous une forme partiellement ou totalement salifiée, avec un contre-ion. Le contre-ion peut être un métal alcalin, tel que le sodium ou le potassium, un alcalino-terreux, tel que le calcium, ou encore un ion ammonium de formule N(R)₄⁺ dans laquelle R, identiques ou différents, représentent un atome d'hydrogène ou un groupe alkyle en C₁-C₄ éventuellement substitué par un atome d'oxygène.

A titres d'exemples de tensioactifs non ioniques, on peut citer, sans intention de s'y limiter :
- les phénols polyalkoxylés (éthoxylés, propoxylés, éthopropoxylés) substitués par au moins un groupe alkyle en C₄-C₂₀, de préférence en C₄-C₁₂, ou substitués par au moins un groupe alkylaryle dont la partie alkyle est en C₁-C₆. Plus particulièrement, le nombre total de motifs alkoxylés est compris entre 2 et 100. A titre d'exemple, on peut citer les mono-, di- ou tri (phényléthyl) phénols polyalkoxylés, ou les nonylphénols polyalkoxylés. Parmi les di- ou tristyrylphenols éthoxylés et/ou propoxylés, sulfatés et/ou phosphatés, on peut citer, le di-(phényl-1 éthyl)phénol éthoxylé, contenant 10 motifs oxyéthylénés, le di-(phényl-1 éthyl)phénol éthoxylé, contenant 7 motifs oxyéthylénés, le di-(phényl-1 éthyl)phénol éthoxylé sulfaté, contenant 7 motifs oxyéthylénés, le tri-(phényl-1 éthyl)phénol éthoxylé, contenant 8 motifs oxyéthylénés, le tri-(phényl-1 éthyl)phénol éthoxylé, contenant 16 motifs oxyéthylénés, le tri-(phényl-1 éthyl)phénol éthoxylé sulfaté, contenant 16 motifs oxyéthylénés, le tri-(phényl-1 éthyl)phénol éthoxylé, contenant 20 motifs oxyéthylénés, le tri-(phényl-1 éthyl)phénol éthoxylé phosphaté, contenant 16 motifs oxyéthylénés.
- les alcools ou les acides gras en C₆-C₂₂, polyalkoxylés (éthoxylés, propoxylés, éthopropoxylés). Le nombre des motifs alkoxylés est compris entre 1 et 60. Le terme acide gras éthoxylé inclut aussi bien les produits obtenus par éthoxylation d'un acide gras par l'oxyde d'éthylène que ceux obtenus par estérification d'un acide gras par un polyéthylèneglycol.
- les triglycérides polyalkoxylés (éthoxylés, propoxylés, éthopropoxylés) d'origine végétale ou animale. Ainsi conviennent les triglycérides issus du saindoux, du suif, de l'huile d'arachide, de l'huile de beurre, de l'huile de graine de coton, de l'huile de lin, de l'huile d'olive, de l'huile de palme, de l'huile de pépins de raisin, de l'huile de poisson, de l'huile de soja, de l'huile de ricin, de l'huile de colza, de l'huile de coprah, de l'huile de noix de coco, et comprenant un nombre total de motifs alkoxylés compris entre 1 et 60. Le terme triglycéride éthoxylé vise aussi bien les produits obtenus par éthoxylation d'un triglycéride par l'oxyde d'éthylène que ceux obtenus par trans-estérification d'un triglycéride par un polyéthylèneglycol.
- les esters de sorbitan éventuellement polyalkoxylés (éthoxylés, propoxylés, éthopropoxylés), plus particulièrement les esters de sorbitol cyclisé d'acides gras de C₁₀ à C₂₀ comme l'acide laurique, l'acide stéarique ou l'acide oléique, et comprenant un nombre total de motifs alkoxylés compris entre 2 et 50.

Des émulsifiants utiles sont notamment les produits suivants, tous commercialisés par Rhodia :
- Soprophor® TSP/724 : tensioactif à base de tristyrylphénol éthopropoxylé
- Soprophor® 796/P : tensioactif à base de tristyrylphénol éthopropoxylé
- Soprophor® CY 8 : tensioactif à base de tristyrylphénol éthoxylé
- Soprophor® BSU : tensioactif à base de tristyrylphénol éthoxylé
- Alkamuls® RC : tensioactif à base d'huile de ricin éthoxylée
- Alkamuls® OR/36 : tensioactif à base d'huile de ricin éthoxylée
- Alkamuls® T/20 : tensioactif à base d'un ester de sorbitan éthoxylé

La formulation comprend avantageusement au moins 2%, de préférence au moins 5%, de préférence au moins 8%, en poids de matière sèche, d'au moins un tensioactif d).

On mentionne que le solvant peut être associé à un tensioactif aromatique et/ou non aromatique.

La formulation phytosanitaire, concentrée, ne comprend de préférence pas des quantités importantes d'eau. Typiquement la teneur en eau est inférieure à 50% en poids, avantageusement inférieure à 25% en poids. Elle sera généralement inférieure à 10% en poids.

La formulation est de préférence un formulation liquide, par exemple sous forme d'un concentré émulsifiable (EC), d'une émulsion concentrée (EW), un concentré soluble (SL), une suspo-émulsion (SE) ou d'une microémulsion (ME). Dans ce cas elle comprend de préférence moins de 500 g/L d'eau, plus préférablement moins de 250 g/L. Elle sera généralement inférieure à 100 g/L.

Les formulations peuvent avantageusement comprendre :
a) de 0,01% à 60%, de préférence de 10% à 50%, du produit phytosanitaire, en poids de matière active,
b) de 10% à 92%, de préférence de 20% à 80%, du composé esteramide de formule (I) selon la présente invention, en poids,
c) de 1 à 88%, de préférence de 2 à 78% en poids, d'au moins un cosolvant ou un autre solvant,
d) de 2% à 60%, de préférence de 5% à 50%, de préférence de 8% à 25%, en poids de matière sèche, d'un tensioactif,
e) de 0 à 30% de préférence de 0 à 20% en poids d'eau.

Il n'est pas exclu de réaliser des formulations solides, par exemple des formulations dans lesquelles un liquide comprenant le produit phytosanitaire solubilisé dans le solvant, est supporté par un minéral et/ou dispersé dans une matrice solide.

La formulation peut bien entendu comprendre d'autres ingrédients (ou "autres additifs") que le produit phytosanitaire actif, le(s) solvant(s), le(s) agent(s) émulsifiant(s) optionnel(s) et l'eau optionnelle. Elle peut notamment comprendre des agents de modification de la viscosité, des agents antimousse, notamment des antimousse siliconés, des agents anti-rebond, des agents anti-lessivage, des charges inertes, notamment des charges minérales, des agents anti-gel, des stabilisants, des colorants, des agents éméthiques, des stickers (promoteurs d'adhésion)...

Notamment les formulations peuvent comprendre des co-solvants ou d'autres solvants c). Les formulations comprennent de tels autres solvants en particuliers lorsque l'esteramide de formule I selon l'invention est utilisé à titre de co-solvant. Les autres solvants ou cosolvants c), sont préférentiellement choisis dans le groupe suivant :
∘ les hydrocarbures aliphatiques, saturés ou insaturés, linéaires ou ramifies, comprenant éventuellement un atome d'halogène, de phosphore, de soufre et/ou d'azote et/ou un groupe fonctionnel,
∘ les hydrocarbures carbocycliques ou hétérocycliques, saturés, insaturés ou aromatiques comprenant éventuellement un atome d'halogène, de phosphore, de soufre et/ou d'azote et/ou un groupe fonctionnel,

De façon encore plus avantageuse, ils sont choisis dans le groupe suivant :
▪ les alcanes, les cycloalcanes et les dérives aromatiques, par exemple les paraffines à chaîne linéaire ou ramifiée comme la « white oil » ou la décaline; les mono, di ou tri alkyl benzènes ou naphtalènes, les composés commercialisés sous la dénomination Solvesso 100, 150, 200 standard et grades ND;
▪ les mono, di ou tri esters aliphatiques, cycloaliphatiques ou aromatiques, par exemple les alcanoates d'alkyle comme l'oléate de méthyle; les alcanoates de benzyle; les benzoates d'alkyle; la gamma butyrolactone; la caprolactone; les esters de glycérol et d'acide citrique; les salicylates d'alkyle; les phtalates; les dibenzoates; les acétoacétates; les acétates d'éther de glycol; le diacétate de dipropylène glycol;
▪ les mono, di or tri phosphates d'alkyle comme par exemple le triéthyl phosphate; le tributyl phosphate;ou le tri-2-éthylhéxylphosphate;
▪ les cétones aliphatiques, cycloaliphatiques, ou aromatiques comme par exemple les dialkyl cétones; les benzyl cétones; la fenchone ; l'acétophénone; la cyclohexanone; les alkyl cyclohexanone;
▪ les alcools aliphatiques, cycloaliphatiques, ou aromatiques comme par exemple les glycols; le 2-éthylhexanol; le cyclohexanol ; les alcools benzyliques; l'alcool tetrahydrofurfurylique ;
▪ les éthers aliphatiques, cycloaliphatiques, ou aromatiques comme par exemple les éthers de glycol, notamment l'éthylène et le propylène glycol, et leurs polymères; l'éther diphénylique; le dipropylène glycol; l'éther monométhylique ou monobutylique; l'éther monobutylique de tripropylène glycol; les alkoxyalcanols; le diméthyl isosorbide;
▪ les acides gras comme par exemple l'acide linoléique; l'acide linolénique; l'acide oléique;
▪ les carbonates comme par exemple le carbonate de propylène ou de butylène; les lactates; les fumarates; les succinates, les adipates, les maléates;
▪ les amides comme par exemple les alkyldiméthylamides, la diméthyldécanoamide;
▪ les alkyl urées;
▪ les amines comme par exemple les alcanolamines, la morpholine; les N-alkyl-pyrrolidones;
▪ la tétraméthyl sulfone;
▪ le diméthyl sulfoxide;
▪ les halogénoalcanes ou les solvants aromatiques halogénés comme par exemple les chloroalcanes ou le chlorobenzène.

Les autres solvants particulièrement préférés sont les alkylbenzènes et naphtalènes, les composés commercialisés sous la dénomination Solvesso 100, 150, 200 standard et grades ND, les alcanolamides et leurs éthers d'alkyle, les acides gras et leurs esters d'alkyle comme par exemple l'oléate de méthyle, les alkyldiméthylamides, les N-alkyl-pyrrolidones, les tri-alkylphosphates, les alcools aliphatiques (linéaires ou ramifiés) et leurs esters, les esters di-basiques, les paraffines (linéaires ou ramifiées) comme la « white oil », les glycols et les éthers de glycol, l'acétophénone.

Peuvent également être présents dans les formulations, des inhibiteurs de cristallisation. Il peut s'agir des solvants mentionnés ci-dessus. Il peut également s'agir d'acides gras ou d'alcools gras non polyalkoxylés, on cite par exemple le produit Alkamuls® OL700 commercialisé par Rhodia, d'alkanolamides, de polymères etc.

Des procédés classiques de préparation de formulations phytosanitaires ou de mélanges de solvants peuvent être mis en oeuvre. On peut opérer par simple mélange des constituants.

La formulation phytosanitaire concentrée est généralement destinée à être répandue sur un champ cultivé ou à cultiver, par exemple un champ de soja, le plus souvent après dilution dans de l'eau, pour obtenir une composition diluée. La dilution est généralement opérée par l'exploitant agricole, directement dans un réservoir ("tank-mix"), par exemple dans le réservoir d'un dispositif destiné à répandre la composition. Il n'est pas exclu que l'exploitant ajoute d'autres produits phytosanitaires, par exemple des fongicides, herbicides, pesticides, insecticides, des fertilisants, des adjuvants... Ainsi, la formulation peut être utilisée pour préparer une composition diluée dans l'eau du produit phytosanitaire actif, par mélange d'au moins une part en poids de formulation concentrée avec au moins 10 parts d'eau, de préférence moins de 10000 parts. Les taux de dilution et les quantités à appliquer sur le champ dépendent généralement du produit phytosanitaire et de la dose souhaitable pour traiter le champ (cela peut être déterminé par l'exploitant agricole).

Les exemples qui suivent illustrent l'invention sans toutefois la limiter.

### Exemples

On décrit la préparation des différents solvants qui seront ensuite mis en oeuvre pour solubiliser certaines matières actives phytosanitaires.

### Exemple 1

### Préparation du composé 1 (2-(diméthylamino)-2-oxoacétate de méthyle) :

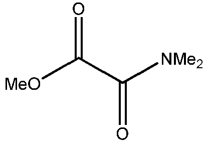

Dans un réacteur de 1 000 mL sont additionnés à 0°C, 360 g (4 mol) de solution de diméthylamine (DMA) à 50% poids dans le méthanol et 43,2 g (0,2 mol) de méthylate de sodium à 25% poids dans le méthanol. Sur ce mélange sont additionnés, à 0-5°C et en 1 heure, 472 g (4 mol) d'oxalate de diméthyle. Le mélange est maintenu sous agitation et à 0°C pendant 2 heures. Le milieu réactionnel est concentré sous vide (Pₘₐₓ = 200 mbar) pour retirer la DMA résiduelle et du méthanol puis une solution d'acide orthophosphorique à 85% (23 g, 0,2 mol) est alors ajoutée. Les sels formés sont filtrés puis le filtrat est distillé sous vide (T_{eb} = 68-70°C, P < 1 mbar) pour récupérer le produit désiré (461 g).

### Exemple 2

### Préparation du composé 2 (2-(diméthylamino)-2-oxoacétate d'éthyle) :

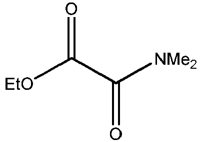

Dans un réacteur de 1 500 mL sont additionnés à 0°C, 360 g (4 mol) de solution de diméthylamine (DMA) à 50% poids dans le méthanol et 44 g (0,2 mol) de méthylate de sodium à 25% poids dans le méthanol. Sur ce mélange sont additionnés, à 0-5°C et en 1 heure, 584 g (4 mol) d'oxalate de diéthyle. Le mélange est maintenu sous agitation et à 0°C pendant 4 heures. Le milieu réactionnel est concentré sous vide (P max = 200 mbar) pour retirer la DMA résiduelle et du méthanol puis une solution d'acide orthophosphorique à 85% (23,3 g, 0,2 mol) est alors ajoutée. Les sels formés sont filtrés puis le filtrat est distillé sous vide (Teb = 77-78°C, P < 1 mbar) pour récupérer le produit désiré (500 g).

### Exemple 3

### Préparation du composé 3 (2-(diméthylamino)-2-oxoacétate de 2-éthyl-hexyle)

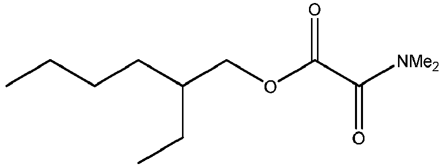

### Mode opératoire en catalyse acide

Dans un réacteur de 1 000 mL, sont mélangés à 20-25°C, le 2-(diméthylamino)-2-oxoacétate de méthyle (composé 1) (390 g, 2,98 mol), le 2-éthyl-hexan-1-ol (464 g, 3,5 mol) et l'acide sulfurique 98% (66 g, 0,67 mol). La température du mélange est portée et maintenue à 80°C pendant 24 heures. L'avancement de la réaction est suivi par analyse par chromatographie en phase gaz. A la fin de la réaction, le méthanol formé est retiré du milieu réactionnel par distillation sous vide réduit (P min = 150 mbar) puis une solution aqueuse d'hydrogénocarbonate de sodium est additionnée au milieu réactionnel de telle sorte que le pH final soit de 7. La phase organique est séparée de la phase aqueuse. Cette dernière est contre-extraite par 500 mL de dichlorométhane. Les phases organiques sont rassemblées puis le milieu réactionnel est concentré pour retirer le dichlorométhane. Au résidu sont additionnés 5 g de carbonate de potassium puis le milieu ainsi obtenu est distillé sous vide partiel (Teb 150°C, P< 1 mbar). Le produit attendu est obtenu avec une pureté supérieure à 98% (578 g).

### Exemple 4

### Préparation du composé 4 (2-(diméthylamino)-2-oxoacétate de n-décyle)

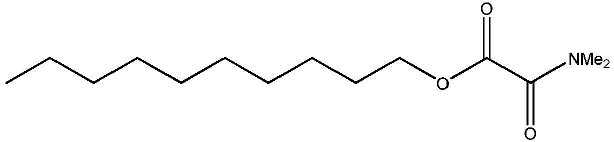

### Mode opératoire en catalyse basique

Dans un réacteur de 250 mL, sont mélangés à 20-25°C, le n-décan-1-ol (114 g, 0,72 mol) et le sodium (2.8 g, 0.12 mol). La température du mélange est portée et maintenue à 50°C pendant 8 heures, jusqu'à disparition totale du solide. Après retour à de la température du milieu réactionnel à 20°C, le 2-(diméthylamino)-2-oxoacétate de méthyle (composé 1) (79 g, 0,6 mol) est additionné et le milieu réactionnel est maintenu sous agitation à 25°C pendant 5 heures. L'avancement de la réaction est suivi par analyse par chromatographie en phase gaz. A la fin de la réaction, une solution d'acide sulfurique 4N (10 mL) à 25°C. Les sels formés sont retirés par filtration. La phase organique est séparée de la phase aqueuse. Cette dernière est contre-extraite par 50 mL de dichlorométhane. Les phases organiques sont rassemblées puis le milieu réactionnel est concentré pour retirer le dichlorométhane. Au résidu sont additionnés 5 g de carbonate de potassium puis le milieu ainsi obtenu est distillé sous vide partiel (Teb 150°C, P< 1 mbar). Le produit attendu est obtenu avec une pureté supérieure à 98% (55 g).

### Mode opératoire en catalyse acide

Dans un réacteur de 1 500 mL, sont mélangés à 20-25°C, le 2-(diméthylamino)-2-oxoacétate de méthyle (composé 1) (398 g, 3 mol), le n-décan-1-ol (567 g, 3,6 mol) et l'acide sulfurique 98% (59 g , 0.6 mol). La température du mélange est portée et maintenue à 120°C pendant 24 heures. L'avancement de la réaction est suivi par analyse par chromatographie en phase gaz. A la fin de la réaction, le méthanol formé est retiré du milieu réactionnel par distillation sous vide réduit (P min = 150 mbar) puis une solution aqueuse d'hydrogénocarbonate de sodium est additionnée au milieu réactionnel de telle sorte que le pH final soit de 7. La phase organique est séparée de la phase aqueuse. Cette dernière est contre-extraite par 500 mL de dichlorométhane. Les phases organiques sont rassemblées puis le milieu réactionnel est concentré pour retirer le dichlorométhane. Au résidu sont additionnés 5 g de carbonate de potassium puis le milieu ainsi obtenu est distillé sous vide partiel (Teb 150°C, P< 1 mbar). Le produit attendu est obtenu avec une pureté supérieure à 98% (633 g).

### Exemple 5

### Préparation du composé 5 (2-(diméthylamino)-2-oxoacétate de cyclohexyle)

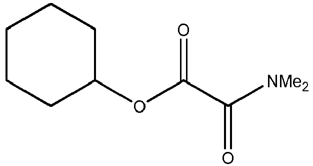

### Mode opératoire en catalyse acide

Dans un réacteur de 1 000 mL, sont mélangés à 20-25°C, le 2-(diméthylamino)-2-oxoacétate de méthyle (composé 1) (393 g, 3 mol), le cyclohexanol (360 g, 3,6 mol) et l'acide sulfurique 98% (66 g, 0,67 mol). La température du mélange est portée et maintenue à 80°C pendant 36 heures. L'avancement de la réaction est suivi par analyse par chromatographie en phase gaz. A la fin de la réaction, le méthanol formé est retiré du milieu réactionnel par distillation sous vide réduit (P min = 150 mbar) puis une solution aqueuse d'hydrogénocarbonate de sodium est additionnée au milieu réactionnel de telle sorte que le pH final soit de 7. La phase organique est séparée de la phase aqueuse. Cette dernière est contre-extraite par 500 mL de dichlorométhane. Les phases organiques sont rassemblées puis le milieu réactionnel est concentré pour retirer le dichlorométhane. Au résidu sont additionnés 5 g de carbonate de potassium puis le milieu ainsi obtenu est distillé sous vide partiel (Teb 115-120°C, P< 1 mbar). Le produit attendu est obtenu avec une pureté supérieure à 98.2% (466 g).

### SOLUBILITÉ DE CERTAINS ACTIFS AGROCHIMIQUES DANS LES SOLVANTS

Les tests suivants ont été effectués :
On réalise des formulations par dilution de divers actifs reportés ci-dessous dans le tableau dans les composés 1 à 4 synthétisés ci-dessus.

### 1) Observation visuelle à 25°C

On note l'aspect de la formulation et on repère éventuellement la présence de cristaux.

### 2) Observation visuelle à 0°C

On place la formulation pendant 7 jours à 0°C et on note l'aspect de la formulation et on repère éventuellement la présence de cristaux (test CIPAC MT39).

### 3) Observation visuelle à 0°C avec nucléation (par introduction dans le liquide d'un cristal de l'actif pur)

On introduit un cristal de la matière active dans la formulation ayant passé 7 jours à 0°C pour nucléation, et on place à nouveau la formulation pendant 7 jours à 0°C. On note l'aspect de la formulation et on repère éventuellement la présence de cristaux ou la croissance du cristal introduit.

Les actifs utilisés sont disponibles commercialement. Lorsque la formation de cristaux d'actif est observée, le terme « Cristal » est indiqué dans le tableau ci-dessous. Dans ce cas, le test suivant n'est pas effectué et le symbole « - » est reporté dans le tableau. Lorsque la solution reste limpide (absence de solide ou de trouble), c'est le terme « limpide » qui est reporté dans le tableau de résultats ci-dessous.

Les résultats sont consignés dans le tableau suivant :

**Tableau (I)**

| Solvant | Actif | Apparence à 25°C | Apparence à 0°C | Apparence à 0°C avec nucléation |
|---|---|---|---|---|
| Composé 1 | Alachlor 48% EC | Limpide | Limpide | Limpide |
| | alpha-Cyperméthrine 1 0% EC | Limpide | Limpide | Limpide |
| | Phenmedipham 16% EC | Limpide | Limpide | Limpide |
| | Propanil 36% EC | Limpide | Limpide | Limpide |
| | Pendimethalin 33% EC | Limpide | Cristal | - |
| | Tebuconazole 25% EC | Limpide | Cristal | - |
| | Trifluralin 40% EC | Limpide | Cristal | - |
| | Difenconazole 25% EC | Limpide | Limpide | Limpide |
| | Dimethoate 40% EC | Limpide | Cristal | - |
| | Oxyfluorfen 22% EC | Limpide | Cristal | - |
| | Propuxur 20% EC | Limpide | Limpide | Limpide |
| | Azoxystrobine 25% EC | Limpide | Cristal | - |
| Composé 2 | Alachlor 48% EC | Limpide | Limpide | Limpide |
| | Chlorpyrifos 40% EC | Limpide | Limpide | Limpide |
| | alpha-Cyperméthrine 1 0% EC | Limpide | Limpide | Limpide |
| | Phenmedipham 16% EC | Limpide | Limpide | Limpide |
| | Propanil 36% EC | Limpide | Limpide | Limpide |
| | Pendimethalin 33% EC | Limpide | Cristal | - |
| | Tebuconazole 25% EC | Limpide | Cristal | - |
| | Trifluralin 40% EC | Limpide | Limpide | Limpide |
| | Difenconazole 25% EC | Limpide | Limpide | Limpide |
| | Dimethoate 40% EC | Limpide | Limpide | Cristal |
| | Oxyfluorfen 22% EC | Limpide | Limpide | Limpide |
| | Propuxur 20% EC | Limpide | Cristal | - |
| | Azoxystrobine 25% EC | Limpide | Cristal | - |
| Composé 3 | Alachlor 48% EC | Limpide | Limpide | Limpide |
| | Chlorpyrifos 40% EC | Limpide | Limpide | Limpide |
| | alpha-Cyperméthrine 1 0% EC | Limpide | Limpide | Limpide |
| | Propanil 36% EC | Limpide | Limpide | Limpide |
| | Pendimethalin 33% EC | Limpide | Cristal | - |
| | Trifluralin 40% EC | Limpide | Limpide | Limpide |
| | Difenconazole 25% EC | Limpide | Limpide | Limpide |
| | Dimethoate 40% EC | Limpide | Cristal | - |
| | Oxyfluorfen 22% EC | Limpide | Limpide | Cristal |
| | Propuxur 20% EC | Limpide | Cristal | - |
| Composé 4 | Alachlor 48% EC | Limpide | Limpide | Limpide |
| | Chlorpyrifos 40% EC | Limpide | Cristal | - |
| | Propanil 36% EC | Limpide | Cristal | - |
| | Trifluralin 40% EC | Limpide | Limpide | Limpide |
| | Difenconazole 25% EC | Limpide | Limpide | Limpide |
| | Oxyfluorfen 22% EC | Limpide | Cristal | - |

Il ressort clairement du tableau ci-dessus que les composés 1 à 4 sont de bons solvants de la plupart des actifs agrochimiques.

## Revendications

1. Utilisation d'un composé esteramide, seul ou en mélange, de formule (I) suivante :
R¹OOC-A-CONR²R³ (I)
où :
- A est une liaison covalente ;
- R¹ est un groupe alkyle comprenant de 1 à 15 atomes de carbone ou un groupe cycloalkyle comprenant de 5 à 8 atomes de carbone,
- R² et R³, identiques ou différents, sont des groupes alkyles comprenant de 1 à 15 atomes de carbone,
à titre de solvant ou co-solvant.

2. Utilisation selon la revendication 1, **caractérisée en ce que** R¹ est choisi parmi les groupes méthyle, éthyle, n-propyle, isopropyle, n-butyle, isobutyle, tertiobutyle, sec-butyle, n-pentyle, isopentyle, sec-pentyle, cyclopentyle, n-hexyle, isohexyle, sec-hexyle, cyclohexyle, méthylcyclohexyle, 2-éthylbutyle, 3-méthylpentyle, n-heptyle, isoheptyle, sec-heptyle, 3-méthylhexyle, 4-méthylhexyle, 1-éthylpentyle, 2-éthylpentyle, 3-éthylpentyle, n-octyle, isooctyle, 3-méthylheptyle, n-nonyle, n-décyle, undécyle, n-dodécyle, tridécyle, tétradécyle et pentadécyle.

3. Utilisation selon l'une quelconque des revendications 1 à 2, **caractérisée en ce que** R² et R³, identiques ou différents, sont choisis parmi les groupes méthyle, éthyle, n-propyle, isopropyle, n-butyle, isobutyle, tertiobutyle, n-pentyle, isoamyle et hexyle.

4. Utilisation selon l'une quelconque des revendications 1 à 3, **caractérisée en ce que** le composé de formule (I) est choisi parmi les composés suivants :
- CH₃OOC-CONMe₂ ;
- CH₃CH₂OOC-CONMe₂,
- CH₃-CH₂-CH₂-CH₂-CH(CH₂CH₃)-CH₂-OOC-CONMe₂ ;
- CH₃-(CH₂)₉-OOC-CONMe₂ ;
- CH₃-(CH₂)₁₁-OOC-CONMe₂ ;
- C₆H₁₁-OOC-CONMe₂ ;
- CH₃OOC-CONEt₂ ;
- CH₃CH₂OOC-CONEt₂ ;
- CH₃-CH₂-CH₂-CH₂-CH(CH₂CH₃)-CH₂-OOC-CONEt₂ ;
- CH₃-(CH₂)₉-OOC-CONEt₂;
- CH₃-(CH₂)₁₁-OOC-CONEt₂
- C₆H₁₁-OOC-CONEt₂.

5. Utilisation selon l'une quelconque des revendications 1 à 4, **caractérisée en ce que** le composé de formule (I) est dans une formulation phytosanitaire, dans une formulation de nettoyage, dans une formulation de décapage, dans une formulation de dégraissage, dans une formulation de lubrifiant, dans une formulation de revêtement, dans une formulation de pigment ou encre, dans une formulation de solubilisation des résines, notamment des résines PVDF ou dans une formulation de nettoyage des « photoresists » ou encore dans une formulation de nettoyage d'écrans à cristaux liquides.

6. Formulation phytosanitaire, comprenant un composé esteramide de formule (I) telle que définie dans l'une quelconque des revendications 1 à 4, en association avec un produit actif.

## Patentansprüche

1. Verwendung einer Esteramid-Verbindung, allein oder im Gemisch, der folgenden Formel (I) :
R¹OOC-A-CONR²R³ (I),
wobei:
- A eine kovalente Bindung ist,
- R¹ eine Alkylgruppe mit 1 bis 15 Kohlenstoffatomen oder eine Cycloalkylgruppe mit 5 bis 8 Kohlenstoffatomen ist,
- R² und R³, die gleich oder verschieden sind, Alkylgruppen mit 1 bis 15 Kohlenstoffatomen sind,
als Lösungsmittel oder Co-Lösungsmittel.

2. Verwendung nach Anspruch 1, **dadurch gekennzeichnet, dass** R¹ aus den Gruppen Methyl, Ethyl, n-Propyl, Isopropyl, n-Butyl, Isobutyl, tert-Butyl, sek-Butyl, n-Pentyl, Isopentyl, sek-Pentyl, Cyclopentyl, n-Hexyl, Isohexyl, sek-Hexyl, Cyclohexyl, Methylcyclohexyl, 2-Ethylbutyl, 3-Methylpentyl, n-Heptyl, Isoheptyl, sek-Heptyl, 3-Methylhexyl, 4-Methylhexyl, 1-Ethylpentyl, 2-Ethylpentyl, 3-Ethylpentyl, n-Octyl, Isooctyl, 3-Methylheptyl, Nonyl, n-Decyl, Undecyl, n-Dodecyl, Tridecyl, Tetradecyl und Pentadecyl ausgewählt ist.

3. Verwendung nach einem der Ansprüche 1 bis 2, **dadurch gekennzeichnet, dass** R² und R³, die gleich oder verschieden sind, aus den Gruppen Methyl, Ethyl, n-Propyl, Isopropyl, n-Butyl, Isobutyl, tert-Butyl, n-Pentyl, Isoamyl und Hexyl ausgewählt sind.

4. Verwendung nach einem der Ansprüche 1 bis 3, **dadurch gekennzeichnet, dass** die Verbindung der Formel (I) aus den folgenden Verbindungen ausgewählt ist :
- CH₃OOC-CONMe₂;
- CH₃CH₂OOC-CONMe₂,
- CH₃-CH₂-CH₂-CH₂-CH(CH₂CH₃)-CH₂-OOC-CONMe₂ ;
- CH₃-(CH₂)₉-OOC-CONMe₂ ;
- CH₃-(CH₂)₁₁-OOC-CONMe₂ ;
- C₆H₁₁OOC-CONMe₂ ;
- CH₃OOC-CONEt₂ ;
- CH₃CH₂OOC-CONEt₂ ;
- CH₃-CH₂-CH₂-CH₂-CH(CH₂CH₃)-CH₂-OOC-CONEt₂ ;
- CH₃-(CH₂)₉-OOC-CONEt₂;
- CH₃-(CH₂)₁₁-OOC-CONEt₂
- C₆H₁₁-OOC-CONEt₂.

5. Verwendung nach einem der Ansprüche 1 bis 4, **dadurch gekennzeichnet, dass** die Verbindung der Formel (I) in einer Pflanzenschutzformulierung, in einer Reinigungsformulierung, in einer Beizformulierung, in einer Entfettungsformulierung, in einer Schmiermittelformulierung, in einer Beschichtungsformulierung, in einer Pigment- oder Tintenformulierung, in einer Solubilisierungsformulierung für Harze, insbesondere PVDF-Harze, oder in einer Reinigungsformulierung für "Photoresiste" oder auch in einer Reinigungsformulierung für Flüssigkristallbildschirme vorliegt.

6. Pflanzenschutzformulierung, umfassend eine Esteramid-Verbindung der Formel (I) nach einem der Ansprüche 1 bis 4 zusammen mit einem Wirkstoff.

## Claims

1. Use of an esteramide compound, alone or as a mixture, of formula (I) below:
R¹OOC-A-CONR²R³ (I)
wherein :
- A is a covalent bond;
- R¹ is an alkyl group comprising from 1 to 15 carbon atoms or a cycloalkyl group comprising from 5 to 8 carbon atoms,
- R² and R³, which may be identical or different, are alkyl groups comprising from 1 to 15 carbon atoms,
as solvent or co-solvent.

2. Use according to Claim 1, **characterized in that** R¹ is selected from methyl, ethyl, n-propyl, isopropyl, n-butyl, isobutyl, tert-butyl, sec-butyl, n-pentyl, isopentyl, sec-pentyl, cyclopentyl, n-hexyl, isohexyl, sec-hexyl, cyclohexyl, methylcyclohexyl, 2-ethylbutyl, 3-methylpentyl, n-heptyl, isoheptyl, sec-heptyl, 3-methylhexyl, 4-methylhexyl, 1-ethylpentyl, 2-ethylpentyl, 3-ethylpentyl, n-octyl, isooctyl, 3-methylheptyl, n-nonyl, n-decyl, undecyl, n-dodecyl, tridecyl, tetradecyl and pentadecyl groups.

3. Use according to either one of Claims 1 and 2, **characterized in that** R² and R³, which may be identical or different, are selected from methyl, ethyl, n-propyl, isopropyl, n-butyl, isobutyl, tert-butyl, n-pentyl, isoamyl and hexyl groups.

4. Use according to any one of Claims 1 to 3, **characterized in that** the compound of formula (I) is chosen from the following compounds :
- CH₃OOC-CONMe₂;
- CH₃CH₂OOC-CONMe₂,
- CH₃-CH₂-CH₂-CH₂-CH(CH₂CH₃)-CH₂-OOC-CONMe₂ ;
- CH₃-(CH₂)₉-OOC-CONMe₂ ;
- CH₃-(CH₂)₁₁-OOC-CONMe₂ ;
- C₆H₁₁-OOC-CONMe₂;
- CH₃OOC-CONEt₂;
- CH₃CH₂OOC-CONEt₂ ;
- CH₃-CH₂-CH₂-CH₂-CH(CH₂CH₃)-CH₂-OOC-CONEt₂ ;
- CH₃-(CH₂)₉-OOC-CONEt₂;
- CH₃-(CH₂)₁₁-OOC-CONEt₂
- C₆H₁₁-OOC-CONEt₂.

5. Use according to any one of Claims 1 to 4, **characterized in that** the compound of formula (I) is in a phytosanitary formulation, in a cleaning formulation, in a stripping formulation, in a degreasing formulation, in a lubricant formulation, in a coating formulation, in a pigment or ink formulation, in a formulation for dissolving resins, in particular PVDF resins, or in a formulation for cleaning "photoresists" or else in a formulation for cleaning liquid crystal screens.

6. Phytosanitary formulation, comprising an esteramide compound of formula (I) as defined in any one of Claims 1 to 4, in combination with an active product.
